# EUROPEAN PATENT SPECIFICATION

(11) **EP 1 858 437 B1**
(45) Date of publication and mention of the grant of the patent: **24.08.2011**
(21) Application number: 05797440.4
(22) Date of filing: 15.09.2005
(51) Int. Cl.: A61F 2/01

(54) **SHAPE MEMORY THIN FILM EMBOLIC PROTECTION DEVICE**
FORMGEDÄCHTNIS-DÜNNFILM-EMBOLIESCHUTZVORRICHTUNG
DISPOSITIF DE PROTECTION EMBOLIQUE A COUCHE MINCE A MEMOIRE DE FORME

(30) Priority: 17.09.2004 US 610898 P
(43) Date of publication of application: 28.11.2007
(73) Proprietor: Nitinol Development Corporation, Fremont, California 94539 (US)
(72) Inventor: DINH, Minh, Q., Union City, CA 94587 (US); RUSSELL, Scott, M., San Jose, CA 95125 (US)
(74) Representative: Belcher, Simon James
(86) International application number: PCT/US2005/033249
(87) International publication number: WO 2006/034074

(56) References cited:
- WO-A-03/015840
- US-A1- 2003 187 495
- US-A1- 2004 098 022
- US-A1- 2004 153 118

## Description

### Background of the Invention

### I. Field of the Invention

The present invention relates to the treatment of vascular disease by either percutaneous angioplasty and stenting or surgery. More particularly, the present invention relates to a system that reduces macro- and micro-embolization during the treatment of vascular disease. Even more particularly, the present invention is directed to a collapsible filter device wherein the filter element comprises a shape memory thin film.

### II. Discussion of the Related Art

A variety of surgical and non-surgical angioplasty procedures have been developed for removing obstructions from blood vessels. Balloon angioplasty utilizes a balloon-tipped catheter which may be inserted within a stenosed region of the blood vessel. By inflation of the balloon, the stenosed region is dilated. Stenting involves the permanent implantation of a metallic scaffold in the area of the obstruction, following balloon dilatation. The stent is often delivered on an angioplasty balloon, and is deployed when the balloon is inflated. Another alternative is the local delivery of medication via an infusion catheter. Other techniques, such as atherectomy, have also been proposed. In atherectomy, a rotating blade is used to shave plaque from an arterial wall. Finally, other techniques such as tissue ablation are sometimes performed to address electrical anomalies in heart rhythm. Surgery involves either removing the plaque from the artery or attaching a graft to the artery so as to bypass the obstructing plaque.

One problem common to all of these techniques is the accidental release of portions of the plaque or thrombus, resulting in emboli which can lodge elsewhere in the vascular system. Such emboli may be dangerous to the patient, and may cause severe impairment of the distal circulatory bed.

Depending upon the vessel being treated, this may result in a stroke, or myocardial infarction or limb ischemia.

Vascular filters or embolism traps for implantation into the vena cava of a patient are well known, being illustrated by, for example, U. S. Patents Nos. 4,727,873 and 4,688,553. Additionally, there is a substantial amount of medical literature describing various designs of vascular filters and reporting the results of the clinical and experimented use thereof. See, for example, the article by Eichelter & Schenk entitled "Prophylaxis of Pulmonary Embolism," Archives of Surgery, Vol. 97, August 1968, pp. 348 et seq. See, also, the article by Greenfield, et al., entitled "A New Intracaval Filter Permitting Continued Flow and Resolution of Emboli", Surgery, Vol. 73, No. 4, pp. 599-606 (1973).

Vascular filters are used, often during a postoperative period, when there is a perceived risk of a patient encountering a pulmonary embolus resulting from clots generated at the surgical site. Typically, the filter is mounted in the vena cava to catch large emboli passing from the surgical site to the lungs.

The vascular filters of the prior art are usually permanently implanted in the venous system of the patient, so that even after the need for the filter has abated, the filter remains in place for the lifetime of the patient, absent surgical removal. U.S. Patent No. 3,952,747 describes a stainless steel filtering device which is permanently implanted transvenously within the inferior vena cava. The filtering device is intended to treat recurrent pulmonary embolism. U.S. Patent No. 4,873,978 describes a catheter device comprising a catheter body having a strainer mounted at its distal end. The strainer is shiftable between an opened configuration where it extends substantially across the blood vessel to entrap passing emboli, and a closed configuration where it retains the captured emboli during removal of the catheter. A mechanism actuable at the proximate end of the catheter body allows selective opening and closing of the strainer. Typically, the strainer is a collapsible cone having an apex attached to a wire running from the distal end to the proximate end of the catheter body.

US-2004/0153118A1 relates to embolic filters. A filter may be made of self-expanding material. One embodiment includes multiple filter layers. An inner filter portion may be supported by an outer filter portion. The inner filter portion may invert on delivery to fit inside the outer filter. The inversion may be accomplished by blood flow in a vessel.

WO-A-03/015840 discloses a filter system according to the preamble of claim 1. The frameless filter has a frustroconical shape.

Permanent implantation may be deemed medically undesirable, but it has been done because vascular filters are implanted in patients primarily in response to potentially life threatening situations. Accordingly, the potential disadvantages of permanent implantations of a vascular filter are often accepted.

Notwithstanding the usefulness of the above-described methods, a need still exists for an apparatus and method for preventing embolization associated with conventional surgery and interventional procedures. In particular, it would be desirable to provide a device which could be located within the vascular system to collect and retrieve portions of plaque and thrombus which have dislodged during the surgery or angioplasty procedure.

### SUMMARY OF THE INVENTION

According to the present invention there is provided a vascular filter system as defined in appended claim 1. The shape memory thin film embolic protection device of the present invention overcomes the disadvantages associated with currently utilized devices.

The present invention provides a vascular filter system useful in the surgical or interventional treatment of vascular disease. Macro- and micro-embolization may occur during percutaneous procedures such as angioplasty, which increases the risk of a minor or major stroke. The system of the present invention for reducing macro- and micro-embolization is very useful in helping to prevent the risk of stroke. However, this system would also be useful in any percutaneous angioplasty, stenting, thrombolysis or tissue ablation procedure, or surgical procedure where embolization is a risk. The vascular filter system of the present invention may decrease embolism while allowing brain, or other distal tissue, perfusion. The filters may be delivered to the location through a guide catheter which may be used for the entire procedure from crossing a lesion to deploying a stent.

The shape memory thin film embolic protection system offers a number of advantages, including easy delivery, compliance to various shaped vessels, low profile and increased radiopacity.

### BRIEF DESCRIPTION OF THE DRAWINGS

The present invention will be apparent upon consideration of the following detailed description, taken in conjunction with the accompanying drawings, in which the reference characters refer to like parts throughout, and in which:
Figure 1 is a diagrammatic representation of a shape memory thin film embolic protection system inside of a vessel in accordance with the present invention.
Figure 2 is a diagrammatic representation of a shape memory thin film embolic protection system in accordance with the present invention.

### DETAILED DESCRIPTION OF THE PREFERRED EMBODIMENTS

The present invention relates to a vascular filter system for use in percutaneous angioplasty and stenting as well as other vascular and non-vascular procedures as detailed herein, and provides for the prevention of distal embolism during vascular procedures. Further, the filter system of the present invention allows for distal perfusion while preventing embolization.

In accordance with one exemplary embodiment, the present invention is directed to a minimally invasive collapsible frameless filter for use in the field of medical procedures on vessels of the circulatory system. However, other uses are possible as the artisan will readily appreciate. Essentially, the frameless filter may be used in any organ in which there is a risk of debris becoming dislodged during a medical procedure. The frameless filter is made of shape memory thin film, via physical vapor deposition or any other suitable process, that shapes like an expanded balloon of a balloon catheter. The device comprises multiple inlet openings at its proximal location to allow blood flow in and outlet openings, for example, a series of pores, at a distal location to filter blood clots and embolic material while allowing blood to exit from the filter. The filter, made from thin film, is a collapsible basket-shaped filter and contains no internal support structure. When the constraining sheath of the catheter is withdrawn during deployment, the shape memory properties of the filter allow it to reform to its programmed shape for capturing of embolic material within a vessel, for example. The flow of the blood assists in the deployment of the device and may enable more complete wall opposition. Essentially, the force of the blood flow facilitates the opening of the filter basket.

The filter device may be introduced into a vascular system in a collapsible configuration and delivered to its location through a guide catheter. When deployed the filter expands across a blood vessel such that blood passing through the blood vessel is delivered through the filter. A proximal inlet portion of the filter has multiple inlet openings to allow blood and embolic material to enter the filter, and a distal outlet portion of the filter has a plurality of small outlet openings (pores) to allow through-passage of blood, while retaining embolic material within the filter.

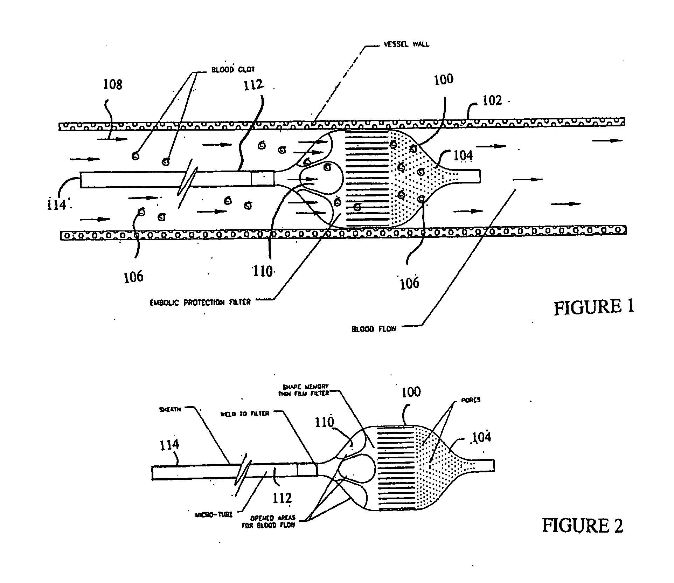

Figure 1 illustrates an exemplary shape memory thin film embolic protection system 100 positioned within a vessel 102. The shape memory thin film embolic protection system 100 comprises a series of pores 104 at its distal end to capture embolic material or blood clots 106 flowing in the blood in the direction of arrows 108. The pores 104 of the thin film capture embolic material but allow blood to pass easily therethrough. The shape memory thin film embolic protection system 100 also comprises inlet openings 110 at its proximal end to allow blood to flow into the filter. The size and shape of the inlet openings 110 may comprise any suitable configuration depending on the application. The shape memory thin film embolic protection system 100 may be connected to the delivery system via any number of means. In the illustrated exemplary embodiment, the thin film section is fastened to a microtube 112 that is operatively associated with a catheter sheath 114. The fastening may be accomplished by any suitable means, including welding. Figure 2 illustrates the same device, but not deployed in the vessel.

The material and design ensure safety and efficacy. The material is designed from a shape memory material. The material may comprise a super elastic or Martensitic shape memory material and, in the preferred embodiment, the material comprises nickel titanium alloy with about 50 to 60 weight percent nickel. The pores of the fabric are designed to capture particulate matter in the size ranging from about 50 µmto about 200 µm.

The shape memory thin film embolic protection system offers a number of advantages. The device is shaped like a non-compliant balloon that will preferably enhance one hundred percent wall opposition. The shape memory thin film with slotted pattern and no internal framework allows an extremely low profile configuration for delivery. The shape memory thin film with slotted pattern and no internal framework allows increased flexibility in the delivery sheath. The outlet openings may be designed to smaller size to allow a smaller capture profile. An increase of the longitudinal length of the device allows increased basket volume. An increase in radiopacity may be achieved by having larger surface areas.

Although shown and described is what is believed to be the most practical and preferred embodiments, it is apparent that departures from specific designs and methods described and shown will suggest themselves to those skilled in the art and may be used without departing from the scope of the invention. The present invention is not restricted to the particular constructions described and illustrated, but should be constructed to cohere with all modifications that may fall within the scope of the appended claims.

## Claims

1. A vascular filter system (100) comprising:
a catheter delivery system, including a sheath (114); and
a collapsible frameless filter comprised of a shape memory, thin film material that expands upon deployment as the sheath (114) is retracted to oppose interior walls of a vessel, the filter having a proximal end with a plurality of openings (110) therein, and a distal end with a plurality of openings (104) therein, wherein blood flow assists the deployment of the filter;
wherein the plurality of openings (104) at the distal end of the collapsible frameless filter permit exit of the blood from the filter while embolic or other particulate materials are captured within the filter; and
wherein the shape memory thin film material further comprises a slotted pattern;
**characterised in that** the plurality of openings (110) at the proximal end of the collapsible frameless filter permit entry of blood into the filter, and **in that** the collapsible frameless filter is shaped like an expanded balloon of a balloon catheter upon deployment.

2. The vascular filter system (100) of claim 1, wherein the size of the plurality of openings (104) at the distal end of the filter determine a capture profile of the filter.

3. The vascular filter system (100) of claim 2, wherein the size of the plurality of openings (104) at the distal end of the filter capture embolic or particulate materials ranging from 50 µm to 200 µm.

4. The vascular filter system (100) of claim 2, wherein the filter is radiopaque.

5. The vascular filter system (100) of claim 4, wherein the shape memory thin film, comprising the filter is biocompatible Nitinol.

6. The vascular filter system (100) of claim 1, wherein the shape memory, thin film material is a super elastic or Martensitic shape memory material.

7. The vascular filter system (100) of claim 7 wherein the filter is comprised of nickel titanium alloy with about 50 to 60 weight percent nickel.

8. The vascular filter system (100) of any one of the preceding claims, further comprising a microtube (112) operatively associated with the sheath (114), wherein the collapsible frameless filter is fastened to the microtube (112), and wherein the slotted pattern is positioned between the plurality of openings (110) at the proximal end and the plurality of openings (104) at the distal end.

## Patentansprüche

1. Vaskularfiltersystem (100), welches Folgendes umfasst:
ein Katheterzuführungssystem, welches eine Hülle (114) aufweist; und
ein zusammenlegbares rahmenloses Filter, welches ein Dünnschichtmaterial mit Formgedächtnis umfasst, welches sich bei der Entfaltung, wenn die Hülle (114) zurückgezogen wird, aufweitet, um sich inneren Wänden eines Gefäßes entgegenzustellen, wobei das Filter ein proximales Ende mit mehreren Öffnungen (110) darin und ein distales Ende mit mehreren Öffnungen (104) darin aufweist, wobei ein Blutfluss die Entfaltung des Filters unterstützt;
wobei die mehreren Öffnungen (104) an dem distalen Ende des zusammenlegbaren rahmenlosen Filters ein Austreten des Blutes aus dem Filter erlauben, während embolische oder andere partikuläre Materialien in dem Filter aufgefangen werden; und
wobei das Dünnschichtmaterial mit Formgedächtnis weiter ein Schlitzmuster umfasst; **dadurch gekennzeichnet, dass** die mehreren Öffnungen (110) an dem proximalen Ende des zusammenlegbaren rahmenlosen Filters einen Eintritt von Blut in das Filter erlauben, und **dadurch**, dass das zusammenlegbare rahmenlose Filter bei der Entfaltung wie ein aufgeweiteter Ballon eines Ballonkatheters geformt ist.

2. Vaskularfiltersystem (100) nach Anspruch 1, wobei die Größe der mehreren Öffnungen (104) an dem distalen Ende des Filters ein Auffangprofil des Filters definieren.

3. Vaskularfiltersystem (100) nach Anspruch 2, wobei die Größe der mehreren Öffnungen (104) an dem distalen Ende des Filters embolische oder partikuläre Materialien in einem Bereich von 50 µm bis 200 µm auffangen.

4. Vaskularfiltersystem (100) nach Anspruch 2, wobei das Filter röntgenstrahlenundurchlässig ist.

5. Vaskularfiltersystem (100) nach Anspruch 4, wobei die Dünnschicht mit Formgedächtnis, welche das Filter umfasst, biokompatibles Nitinol ist.

6. Vaskularfiltersystem (100) nach Anspruch 1, wobei das Dünnschichtmaterial mit Formgedächtnis ein superelastisches oder martensitisches Formgedächtnismaterial ist.

7. Vaskularfiltersystem (100) nach Anspruch 7, wobei das Filter eine Nickel-TitanLegierung mit etwa 50 bis 60 Gewichtsprozent Nickel umfasst.

8. Vaskularfiltersystem (100) nach einem der vorhergehenden Ansprüche, welches weiter einen Mikrotubus (112) in Wirkverbindung mit der Hülle (114) umfasst, wobei das zusammenlegbare rahmenlose Filter an dem Mikrotubus (112) befestigt ist, und wobei das Schlitzmuster zwischen den mehreren Öffnungen (110) an dem proximalen Ende und den mehreren Öffnungen (104) an dem distalen Ende positioniert ist.

## Revendications

1. Système de filtrage vasculaire (100) comprenant :
> un système de mise en place de cathéter comprenant une gaine (114) ; et
> un filtre sans cadre repliable constitué d'un matériau en film fin à mémoire de forme qui se déplie par déploiement lorsque la gaine (114) est rétractée pour s'opposer aux parois intérieures d'un vaisseau, le filtre ayant une extrémité proximale doté d'une pluralité d'oùvertures (110) dans celui-ci et une extrémité distale dotée d'une pluralité d'ouvertures (104) dans celui-ci, le flux sanguin aidant au déploiement du filtre ;
la pluralité d'ouvertures (104) à l'extrémité distale du filtre sans cadre repliable permettant la sortie du sang du filtre, les matériaux emboliques ou autres matériaux particulaires étant capturés dans le filtre ; et
le matériau en film fin à mémoire de forme comprenant en outre une configuration fendue ;
**caractérisé en ce que** la pluralité d'ouvertures (110) à l'extrémité proximale du filtre sans cadre repliable permet l'entrée du sang dans le filtre et **en ce que** le filtre sans cadre repliable est formé comme un ballonnet déployé d'un cathéter à ballonnet lors du déploiement.

2. Système de filtrage vasculaire (100) selon la revendication 1, dans lequel la taille de la pluralité d'ouvertures (104) à l'extrémité distale du filtre détermine un profil de capture du filtre.

3. Système de filtrage vasculaire (100) selon la revendication 2, dans lequel la taille de la pluralité d'ouvertures (104) à l'extrémité distale du filtre capture des matériaux emboliques ou particulaires de l'ordre de 50 µm à 200 µm.

4. Système de filtrage vasculaire (100) selon la revendication 2, dans lequel le filtre est radio-opaque.

5. Système de filtrage vasculaire (100) selon la revendication 4, dans lequel le film fin à mémoire de forme constituant le filtre est en Nitinol biocompatible.

6. Système de filtrage vasculaire (100) selon la revendication 1, dans lequel le matériau en film fin à mémoire de forme est un matériau à mémoire de forme super-élastique ou martensitique.

7. Système de filtrage vasculaire (100) selon la revendication 7, dans lequel le filtre est constitué d'un alliage de nickel titane comprenant environ 50 à 60 % en poids de nickel.

8. Système de filtrage vasculaire (100) selon l'une quelconque des revendications précédentes, comprenant en outre un microtube (112) associé fonctionnellement à la gaine (114), le filtre sans cadre repliable étant fixé au microtube (112) et la configuration fendue étant placée entre la pluralité d'ouvertures (110) à l'extrémité proximale et la pluralité d'ouvertures (104) à l'extrémité distale.
